# EUROPEAN PATENT APPLICATION

(11) **EP 4 552 644 A1**
(43) Date of publication of application: **14.05.2025**
(21) Application number: 23834952.6
(22) Date of filing: 07.07.2023
(51) Int. Cl.: A61K 36/73, A61Q 19/08, A61P 17/18, A61P 39/06, A61K 133/00

(54) **USE OF PRUNUS LANNESIANA EXTRACT IN PREPARATION OF DRUG FOR IMPROVING SKIN CONDITION OR BASAL METABOLIC RATE**

(30) Priority: 08.07.2022 US 202263359204 P
(71) Applicant: TCI Co., Ltd., Taipei 11494 (TW)
(72) Inventor: LIN, Yung-Hsiang, 11494 Taipei Taiwan (TW); LIN, Hsiao-Nai, 11494 Taipei Taiwan (TW); LIN, Huan-You, 11494 Taipei Taiwan (TW)
(74) Representative: Viering, Jentschura & Partner mbB Patent- und Rechtsanwälte
(86) International application number: PCT/CN2023/106377
(87) International publication number: WO 2024/008187

(57) **Abstract**

Use of a *Prunus lannesiana* extract in improving skin condition and basal metabolic rate is about the use of the *Prunus lannesiana* extract for preparing a composition for improving skin condition or basal metabolic rate. The *Prunus lannesiana* extract is obtained by drying *Prunus lannesiana* and extracting the dried *Prunus lannesiana* with a solvent at a weight ratio of the solvent to the *Prunus lannesiana* of (20-40): 1.

## Description

### BACKGROUND

### Technical Field

The present invention relates to use of a *Prunus lannesiana* extract and particularly relates to the use of the *Prunus lannesiana* extract for preparing a composition for improving a skin condition or a basal metabolic rate.

### Related Art

Since the development of organic and/or natural dietary concepts, biotechnology companies and food companies have actively invested in the research and development of related products of natural plants. In order to scientifically verify the benefits of plant-related products to health, the analysis of active ingredients and the evaluation of efficacy of plants become key projects for product development.

*Prunus lannesiana* is one of the only two edible cherry blossom variety in China. Therefore, the biotechnology companies and food companies actively study the analysis of active ingredients and the evaluation of efficacy of the *Prunus lannesiana* so as to develop it into related products.

### SUMMARY

In some embodiments, use of a *Prunus lannesiana* extract for preparing a composition for improving a skin condition is provided. The *Prunus lannesiana* extract is obtained by drying *Prunus lannesiana* and extracting the dried *Prunus lannesiana* with a solvent at a weight ratio of the solvent to the *Prunus lannesiana* of (20-40): 1.

In some embodiments, the *Prunus lannesiana* extract improves a skin collagen density.

In some embodiments, the *Prunus lannesiana* extract reduces skin wrinkles and/or fine lines.

**In** some embodiments, the *Prunus lannesiana* extract relieves skin redness.

**In** some embodiments, the *Prunus lannesiana* extract slows down skin aging or oxidation.

**In** some embodiments, the *Prunus lannesiana* extract decreases skin roughness, improves stability of skin condition, improves skin tightness, relieves sallow skin, improves glossy and bright skin, improves tender and smooth skin, relieves skin sensitivity or improves skin moisture.

**In** some embodiments, use of a *Prunus lannesiana* extract for preparing a composition for improving a basal metabolic rate is provided. The *Prunus lannesiana* extract is obtained by drying *Prunus lannesiana* and extracting the dried *Prunus lannesiana* with a solvent at a weight ratio of the solvent to the *Prunus lannesiana* of (20-40): 1.

In some embodiments, the *Prunus lannesiana* extract improves activity of mitochondria.

In some embodiments, the *Prunus lannesiana* extract prevents and/or reduces DNA damage.

In some embodiments, the *Prunus lannesiana* extract improves an antioxidant activity.

In some embodiments, the *Prunus lannesiana* extract reduces generation of advanced glycation end products.

In summary, use of the *Prunus lannesiana* extract of any embodiments in improving a skin condition or a basal metabolic rate relates to the use of the *Prunus lannesiana* extract for preparing a composition for improving a skin condition or a basal metabolic rate, thereby providing the composition capable of improving the skin condition or the basal metabolic rate on an individual when administered to the individual. In other words, the composition has functions of improving the skin condition or the basal metabolic rate. In some embodiments, the composition prepared from the *Prunus lannesiana* extract further has one or more of the following functions: improving skin collagen density, reducing skin wrinkles and/or fine lines, relieving skin redness, slowing down skin aging or oxidation, decreasing skin roughness, improving stability of skin condition, improving skin tightness, relieving sallow skin, improving glossy and bright skin, improving tender and smooth skin, relieving skin sensitivity, improving skin moisture, improving activity of mitochondria, preventing and/or reducing DNA damage, improving antioxidant activity, and reducing generation of advanced glycation end products.

### BRIEF DESCRIPTION OF THE DRAWINGS

FIG. 1 is a bar chart showing the relative ROS level after treated by a *Prunus lannesiana* extract in accordance with some embodiments of the present disclosure.
FIG. 2 is a bar chart showing the relative activity of mitochondria after treated by *a Prunus lannesiana* extract in accordance with some embodiments of the present disclosure.
FIG. 3 is a bar chart showing the relative content of pyruvate after treated by a *Prunus lannesiana* extract in accordance with some embodiments of the present disclosure.
FIG. 4 is a bar chart showing the relative amount of DNA damage after treated by *a Prunus lannesiana* extract in accordance with some embodiments of the present disclosure.
FIG. 5 is a bar chart showing the concentration of advanced glycation end products in blood at week 0 and week 4 after ingesting a composition including a *Prunus lannesiana* extract in accordance with some embodiments of the present disclosure.
FIG. 6 is a bar chart showing the relative skin collagen density at week 0 and week 4 after ingesting a composition including a *Prunus lannesiana* extract in accordance with some embodiments of the present disclosure.
FIG. 7 is a bar chart showing the relative skin texture degree at week 0 and week 4 after ingesting a composition including a *Prunus lannesiana* extract in accordance with some embodiments of the present disclosure.
FIG. 8 shows photographs of skin texture of one of 10 subjects at week 0 and week 4 after ingesting a composition including a *Prunus lannesiana* extract in accordance with some embodiments of the present disclosure.
FIG. 9 is a bar chart showing the relative skin wrinkle degree at week 0 and week 4 after ingesting a composition including a *Prunus lannesiana* extract in accordance with some embodiments of the present disclosure.
FIG. 10 shows photographs of fine lines around the eyes of one of 10 subjects at week 0 and week 4 after ingesting a composition including a *Prunus lannesiana* extract in accordance with some embodiments of the present disclosure.
FIG. 11 is a bar chart showing the relative skin redness degree at week 0 and week 4 after ingesting a composition including a *Prunus lannesiana* extract in accordance with some embodiments of the present disclosure.
FIG. 12 shows photographs of skin redness of one of 10 subjects at week 0 and week 4 after ingesting a composition including a *Prunus lannesiana* extract in accordance with some embodiments of the present disclosure.
FIG. 13 is a horizontal bar chart showing the percentage of subjects of a questionnaire on self-assessment of skin condition in a human subject test.
FIG. 14 is a pie chart showing the percentage of subjects of a questionnaire on self-assessment of efficacy satisfaction degree in a human subject test.

### DETAILED DESCRIPTION

As used herein, the unit of content "%" used in reference to content generally refers to weight percentage.

In some embodiments, *a Prunus lannesiana* extract is obtained by performing an extraction process by using *Prunus lannesiana* as a raw material. The extraction process mainly comprises extracting *Prunus lannesiana* by a solvent to dissolve effective components in the *Prunus lannesiana* into the solvent.

In some embodiments, the *Prunus lannesiana* subjected to the extraction is *Prunus lannesiana* flowers. In some embodiments, the *Prunus lannesiana* flowers subjected to the extraction are flowers at a blooming stage. In some embodiments, the *Prunus lannesiana* flowers subjected to the extraction comprise petals, calyces, stamens and pistils.

In some embodiments, the *Prunus lannesiana* subjected to the extraction may be intact flowers not subjected to physical pretreatment (i.e., petals, calyces, stamens and pistils are not separated and their sizes are not physically pretreated), or physically pretreated and broken into fine-sized pieces, granules or powders. The physical pretreatment used may include at least one of coarse crushing, chopping, shearing, mashing and grinding.

In some embodiments, the *Prunus lannesiana* subjected to the extraction may be freshly collected, dried, and/or frozen flowers. For example, in the extraction process, the dried *Prunus lannesiana* is extracted with a solvent.

In some embodiments, step of performing the extraction process comprises extracting the *Prunus lannesiana* with water at 70°C-90°C for 60-80 minutes to obtain a primary extract. For example, the *Prunus lannesiana* can be soaked in water at 85±5°C for 60 minutes to dissolve effective components in the *Prunus lannesiana* into the water to obtain the primary extract.

In some embodiments, in the extraction process, the solvent is water, the raw material is Prunus *lannesiana,* and the weight ratio of the solvent to the raw material during an initial mixing is (20-40): 1. For example, the weight ratio of the water to the *Prunus lannesiana* is 35:1.

In some embodiments, the primary extract may be further filtered to remove solids such as *Prunus lannesiana* extracted by water during the extraction process to obtain a filtrate. For example, the primary extract may be filtered through a 400-mesh filter to remove solids, and the filtered filtrate is collected.

In some embodiments, the filtrate may be further concentrated during the extraction process to obtain a concentrate. In some embodiments, the filtrate may be concentrated under reduced pressure at 40°C-55°C to obtain the concentrate. For example, the filtrate may be concentrated under reduced pressure at 60±5°C. In some embodiments, the duration for concentration can be determined by, but is not limited to, the degrees Brix of the concentrate. In light of the previous embodiment, the degrees Brix of the resulting concentrate is 10±0.5° Bx. In other words, the filtrate can be concentrated at 60±5°C under reduced pressure until the filtrate after the reduced pressure concentration has 10±0.5° Bx. The filtrate after the reduced pressure concentration at this time is the concentrate.

In some embodiments, the primary extract may be firstly concentrated to form a concentrate with the reduced liquid volume during the extraction process. Then the concentrate is filtered to remove solids from the concentrate and the filtered filtrate is collected.

In some embodiments, the primary extract is only concentrated or filtered during the extraction process.

It should be understood that the primary extract, filtrate, or concentrate obtained by the extraction process can be used as a *Prunus lannesiana* extract according to the actual requirements.

In some embodiments, the *Prunus lannesiana* extract has the ability of improving a skin condition. Therefore, the *Prunus lannesiana* extract is suitable for preparing a composition for improving the skin condition.

In some embodiments, the *Prunus lannesiana* extract has the ability of improving a skin collagen density. In other words, when administered to an individual, the *Prunus lannesiana* extract can improve the skin collagen density of the individual. Therefore, the *Prunus lannesiana* extract is suitable for preparing a composition for improving the skin collagen density.

In some embodiments, the *Prunus lannesiana* extract has the ability of reducing skin wrinkles and/or fine lines. In other words, when administered to an individual, the *Prunus lannesiana* extract can reduce skin wrinkles and/or fine lines of the individual. Therefore, the *Prunus lannesiana* extract is suitable for preparing a composition for reducing skin wrinkles and/or fine lines.

In some embodiments, the *Prunus lannesiana* extract has the ability of relieving skin redness. In other words, when administered to an individual, the *Prunus lannesiana* extract can relieve skin redness of the individual. Therefore, the *Prunus lannesiana* extract is suitable for preparing a composition for relieving skin redness.

In some embodiments, the *Prunus lannesiana* extract has the ability of slowing down skin aging or oxidation. In other words, when administered to an individual, the *Prunus lannesiana* extract can slow down skin aging or oxidation of the individual. Therefore, the *Prunus lannesiana* extract is suitable for preparing a composition for slowing down skin aging or oxidation.

In some embodiments, the *Prunus lannesiana* extract has the ability of decreasing skin roughness, improving stability of skin condition, improving skin tightness, relieving sallow skin, improving glossy and bright skin, improving tender and smooth skin, relieving skin sensitivity or improving skin moisture. In other words, when administered to an individual, the *Prunus lannesiana* extract can decrease skin roughness, improve stability of skin condition, improve skin tightness, relieve sallow skin, improve glossy and bright skin, improve tender and smooth skin, relieve skin sensitivity or improve skin moisture of the individual. Therefore, the *Prunus lannesiana* extract is suitable for preparing a composition for decreasing skin roughness, improving stability of skin condition, improving skin tightness, relieving sallow skin, improving glossy and bright skin, improving tender and smooth skin, relieving skin sensitivity or improving skin moisture.

In some embodiments, the *Prunus lannesiana* extract has the ability of improving a basal metabolic rate. Therefore, the *Prunus lannesiana* extract is suitable for preparing a composition for improving the basal metabolic rate.

In some embodiments, the *Prunus lannesiana* extract has the ability of improving activity of mitochondria. In other words, when administered to an individual, the *Prunus lannesiana* extract can improve activity of mitochondria of the individual. Therefore, the *Prunus lannesiana* extract is suitable for preparing a composition for improving activity of mitochondria.

In some embodiments, the *Prunus lannesiana* extract has the ability of preventing and/or reducing DNA damage. In other words, when administered to an individual, the *Prunus lannesiana* extract can prevent and/or reduce DNA damage of the individual. Therefore, the *Prunus lannesiana* extract is suitable for preparing a composition for preventing and/or reducing DNA damage.

In some embodiments, the *Prunus lannesiana* extract has the ability of improving antioxidant activity. In other words, when administered to an individual, the *Prunus lannesiana* extract can improve antioxidant activity of the individual. Therefore, the *Prunus lannesiana* extract is suitable for preparing a composition for improving antioxidant activity.

In some embodiments, the *Prunus lannesiana* extract has the ability of reducing generation of advanced glycation end products. In other words, when administered to an individual, the *Prunus lannesiana* extract can reduce generation of advanced glycation end products of the individual. Therefore, the *Prunus lannesiana* extract is suitable for preparing a composition for reducing generation of advanced glycation end products.

In some embodiments, the individual may be human.

In some embodiments, in the composition, the amount of the *Prunus lannesiana* extract consumed per day is 0.2 grams to 0.7 grams. In some embodiments, in the composition, the amount of the *Prunus lannesiana* extract consumed per day is 0.2 grams. In some other embodiments, in the composition, the amount of the *Prunus lannesiana* extract consumed per day is 0.7 grams.

In some embodiments, the prepared composition may be a pharmaceutical composition, a non-medical purpose edible composition, a cosmetic composition or a skin-care product composition.

In some embodiments, when the composition is a pharmaceutical composition, the pharmaceutical composition contains an effective content of a *Prunus lannesiana* extract. The pharmaceutical composition is formulated into enteral, parenteral, oral or topical administration dosages by using techniques well known to those skilled in the art.

In some embodiments, the enteral or oral administration dosage may be, but is not limited to, a troche, a tablet, a lozenge, a pill, a capsule, a dispersible powder or a granule, a solution, a suspension, an emulsion, a syrup, an elixir, a slurry or the like.

In some embodiments, the parenteral or topical administration dosage may be, but is not limited to, an injection (e.g., a sterile aqueous solution or a dispersion), a sterile powder, an external preparation or the like.

In some embodiments, the administration mode of the injection may be, but is not limited to, an intraperitoneal injection, a subcutaneous injection, an intraepidermal injection, an intradermal injection, an intramuscular injection, an intravenous injection or an intralesional injection.

In some embodiments, the pharmaceutical composition containing an effective content of a *Prunus lannesiana* extract may further contain a pharmaceutically acceptable carrier that is widely used in pharmaceutical manufacturing technology. In some embodiments, the pharmaceutically acceptable carrier may be one or more of the following carriers: a solvent, a buffer, an emulsifier, a suspending agent, a decomposer, a disintegrating agent, a dispersing agent, a binding agent, an excipient, a stabilizing agent, a chelating agent, a diluent, a gelling agent, a preservative, a wetting agent, a lubricant, an absorption delaying agent, a liposome and the like. The types and amounts of the carriers selected for use are within the professional literacy and routine technology scopes of those skilled in the art. The solvent as the pharmaceutically acceptable carrier may be water, normal saline, phosphate buffered saline (PBS) or an alcohol containing aqueous solution.

In some embodiments, the pharmaceutical composition containing an effective content of a *Prunus lannesiana* extract may be formulated into an external preparation suitable for topical application to skin by using techniques well known to those skilled in the art. In some embodiments, the external preparation includes, but is not limited to, an emulsion, a gel, an ointment, a cream, a patch, a liniment, a powder, an aerosol, a spray, a lotion, a serum, a paste, a foam, a drop, a suspension, a salve and a bandage.

In some embodiments, when the pharmaceutical composition is the external preparation, the pharmaceutical composition can be prepared by mixing an effective content of *a Prunus lannesiana* extract with a base well known to those skilled in the art.

In some embodiments, the base may include one or more selected from the following additives: water, alcohols, glycol, hydrocarbons (such as petroleum jelly and white petrolatum), waxes (such as paraffin and yellow wax), preserving agents, antioxidants, surfactants, absorption enhancers, stabilizing agents, gelling agents (such as carbopol^{®}974P, microcrystalline cellulose and carboxymethylcellulose), active agents, humectants, odor absorbers, fragrances, pH adjusting agents, chelating agents, emulsifiers, occlusive agents, emollients, thickeners, solubilizing agents, penetration enhancers, anti-irritants, colorants, propellants and the like. The selection and amounts of these additives are within the professional literacy and routine technology scopes of those skilled in the art.

In some embodiments, when the composition is a non-medical purpose edible composition, the edible composition contains a specific content of a *Prunus lannesiana* extract. The edible composition is in a form of a powder, a granule, a solution, a colloid or a paste.

In some embodiments, the edible composition containing a *Prunus lannesiana* extract can be a food product or a food additive.

In some embodiments, the food product containing a *Prunus lannesiana* extract may be beverages, fermented foods, bakery products, health foods, dietary supplements or the like. In some embodiments, the food product containing a *Prunus lannesiana* extract may further comprise an adjuvant. For example, the adjuvant may be maltodextrin, malic acid, sucralose, citric acid, a fruit flavor, a honey flavor, stevioside or a combination thereof. The types and amounts of the adjuvant selected for use are within the professional literacy and routine technology scopes of those skilled in the art.

In some embodiments, the food additive containing a *Prunus lannesiana* extract may be a seasoning, a sweetener, a spice, a pH adjusting agent, an emulsifier, a color additive, a stabilizer or the like.

In some embodiments, the composition is a cosmetic composition or a skin-care product composition. In other words, the cosmetic composition or the skin-care product composition contains an effective content of a *Prunus lannesiana* extract.

In some embodiments, the cosmetic composition or the skin-care product composition containing a *Prunus lannesiana* extract may be in any of the following forms: a toner, a gel, a jelly mask, a mudpack, a lotion, a cream, a lipstick, a foundation, a pressed powder, a loose powder, a cleansing oil, a makeup removing lotion, a facial cleanser, a body wash, a shampoo, a hair conditioner, a sunscreen lotion, a hand cream, a nail polish, a perfume, an essence and a facial mask.

In some embodiments, the cosmetic composition or the skin-care product composition containing a *Prunus lannesiana* extract may further contain an external product acceptable ingredient as needed. In some embodiments, the external product acceptable ingredient may be, for example, an emulsifier, a penetration enhancer, a softener, a solvent, an excipient, an antioxidant or a combination thereof.

Unless otherwise specified in the following examples, the experimental steps are performed at room temperature (25°C-30°C) and atmospheric pressure (1 atm).

### Example I Preparation of Prunus lannesiana extract

A. Materials:
   1. Whole flowers of *Prunus lannesiana* (produced in China) containing petals, calyces, stamens and pistils.
   2. Secondary water, also called reverse osmosis (RO) water or secondary distilled water, which is hereinafter referred to as "water".
B. Preparation process:
   1. After whole flowers of *Prunus lannesiana* were dried at 40-80°C, the dried whole flowers of *Prunus lannesiana* were ground to form *Prunus lannesiana* powder.
   2. After water was heated to 85±5°C, the *Prunus lannesiana* powder was put and soaked in the water at 85±5°C for 60 min to obtain a primary extract containing solids. Here the weight ratio of the *Prunus lannesiana* powder to the water was 1:35.
   3. The primary extract cooled to 65±5°C was filtered through a 400-mesh filter to remove solids (i.e. the extracted *Prunus lannesiana* powder) to obtain a filtrate.
   4. After the temperature of a concentrator (model: Rotavapor R-100; brand: BUCHI) was set at 60±5°C, the filtrate was concentrated under reduced pressure by using the concentrator. When the degrees Brix of the filtrate was concentrated to 10±0.5° Bx, the concentration was stopped to obtain a concentrate.
   5. 1% malic acid was added into the concentrate to adjust the pH value of the concentrate to the pH of 3.2±0.5 to obtain a *Prunus lannesiana* extract.

### Example II Oxidation resistance test

A. Materials and instruments:
   1. Cell line: human skin fibroblasts were purchased from the Bioresource Collection and Research Center (BCRC) with the cell number 60153 and hereinafter referred to as CCD-966SK cells.
   2. Cell culture medium: minimum essential medium (MEM) (purchased from Gibco with the product number 11095080) containing 10% fetal bovine serum (purchased from Gibco with the product number 10437-028), 1% penicillin-streptomycin (purchased from Gibco with the product number 15140122), 1 mM sodium pyruvate (purchased from Gibco with the product number 11360-070), 1.5 g/L of sodium bicarbonate (purchased from Sigma with the product number S5761-500G) and 0.1 mM non-essential amino acids. The 0.1 mM non-essential amino acids were prepared by diluting an MEM non-essential amino acid solution (100X) (purchased from Gibco with the product number 11140050) with the MEM at 99 times by volume of the solution.
   3. Trypsin: prepared by diluting 10X trypsin (purchased from Gibco with the product number 15400-054) with DPBS at 9 times by volume of the 10X trypsin.
   4. 100 mM H₂O₂ solution: prepared from H₂O₂ (purchased from Sigma with the product number 1.08600) and DPBS.
   5. DCFH-DA acting reagent: prepared from DCFH-DA (2',7'-dichlorodihydrofluorescein diacetate) (purchased from Sigma with the product number SI-D6883-50MG) and DMSO (purchased from J.T. Baker with the product number 9224-03).
   6. Flow cytometer, purchased from BD company.
B. Test process:
   1. CCD-966SK cells were inoculated at the density of 2×10⁵ per well in a 6-well culture plate containing 2 mL of the cell culture medium per well and cultured at 37°C for 24 hours. Here the CCD-966SK cells were divided into three test groups, namely a blank group, a control group and an experimental group respectively. Each group was established in triplicate.
   2. After 24 hours of the culture and attachment of the CCD-966SK cells, the cell culture medium in each group was replaced with an experimental culture medium. The experimental culture medium of the blank group and the control group was the cell culture medium free of a sample; and the experimental culture medium of the experimental group was the cell culture medium containing 0.25% (m/v, mass/volume percent, mass of solute per unit volume of solution) of the *Prunus lannesiana* extract prepared in example I. Next the cells in each group reacted at 37°C for 1 hour.
   3. After 1 hour of the reaction, a DCFH-DA acting reagent was added to each group to the final concentration of 5 µg/mL and the cells in each group reacted at 37°C for 15 minutes.
   4. After 15 minutes of the reaction, a 100 mM H₂O₂ solution was added in the control group and the experimental group to the final concentration of 1 mM, and the cells in each group reacted at 37°C for 1 hour.
   5. The experimental culture medium in each group after the reaction was removed and the cells were rinsed 2 times with DPBS.
   6. After the rinsing, 200 µL of trypsin was added to each well and reacted for 5 minutes in the dark. After the reaction, the cell culture medium was added to terminate the reaction. Then the suspended cells and the cell culture medium in each well were collected into corresponding centrifuge tubes and each centrifuge tube was centrifuged to precipitate the cells.
   7. After a supernatant in the centrifuge tube of each group was removed and the precipitated cells were washed with DPBS 1 time, 1 mL of DPBS was added to the centrifuge tube of each group to resuspend the cells to form a cell suspension.
   8. After the parameter of an excitation light of the flow cytometer was set to 450-490 nm and the parameter of an emission light was set to 510-550 nm, green fluorescence signals of each group were detected by the flow cytometer.
C. Test results:
   The relative reactive oxygen species (ROS) level of all groups was calculated according to the following formula: relative ROS level (%) = (green fluorescence signal of each group/green fluorescence signal of blank group)×100%.

Statistically significant differences between the test results of the blank group and each of the other groups, and between the control group and each of the other groups were obtained by a statistical analysis of a student t-test. In the figure, "#" represented a p-value less than 0.05 when compared to the blank group, "##" represented a p-value less than 0.01 when compared to the blank group, and "###" represented a p-value less than 0.001 when compared to the blank group. Also, in the figure, "*" represented a p-value less than 0.05 when compared to the control group, "**" represented a p-value less than 0.01 when compared to the control group, and "***" represented a p-value less than 0.001 when compared to the control group.

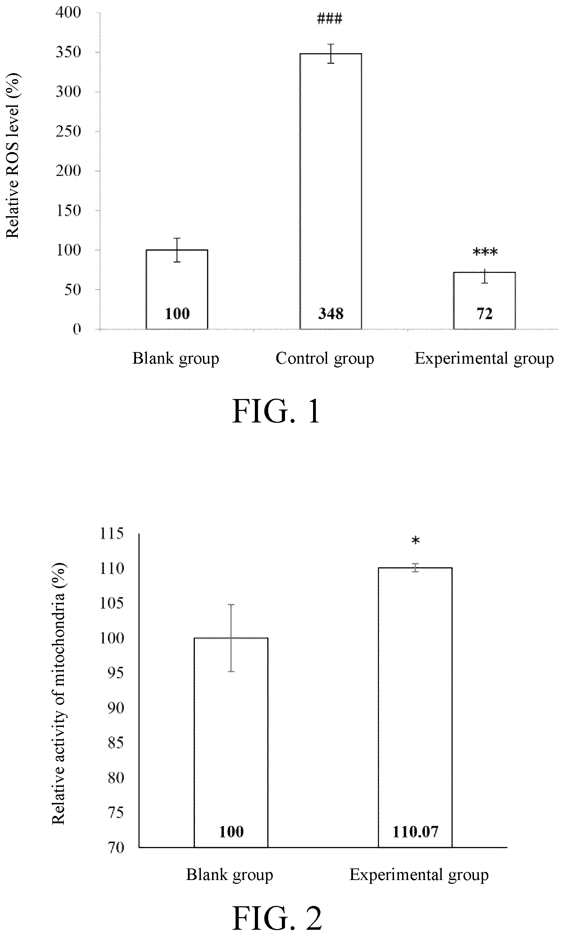

Refer to FIG. 1. The cells in the blank group were not treated with a sample and were not stimulated with H₂O₂, such that the test results in the blank group represented the performance of the cells under normal physiological metabolism. Here when the relative ROS level in the blank group was set to 100%, the relative ROS level in the control group was 348%, and the relative ROS level in the experimental group was 72%. That is, compared to the blank group. the relative ROS level in the control group was significantly increased by about 248% after the stimulation with H₂O₂ to the cells in the control group. Compared to the control group, the relative ROS level in the experimental group was significantly reduced by about 79.3% after adding the *Prunus lannesiana* extract and the stimulation with H₂O₂ to the cells in the experimental group. Compared to the blank group, the relative ROS level in the experimental group was reduced by about 28%.

It can be seen from the test results that the *Prunus lannesiana* extract can significantly reduce the ROS level of skin cells caused by H₂O₂. H₂O₂ induces the cells to undergo aerobic metabolism, thereby producing ROS. The ROS in the cells attack macromolecules such as proteins, nucleic acids, lipids and so on, thereby causing cell damage and triggering cell apoptosis. In other words, it is proved by the test results that the *Prunus lannesiana* extract has the effect of improving the oxidation resistance of skin cells, so as to relieve cell aging caused by oxidation pressure and slow down skin aging or oxidation, and further improve a skin condition. The *Prunus lannesiana* extract has the effect of improving antioxidant activity.

### Example III Test for activity of mitochondria

A. Materials and instruments:
   1. Cell line: human skin fibroblasts were purchased from the BCRC with the cell number 60153 and hereinafter referred to as CCD-966SK cells.
   2. Cell culture medium: minimum essential medium (MEM) (purchased from Gibco with the product number 11095080), containing 10% fetal bovine serum (purchased from Gibco with the product number 10437-028), 1% penicillin-streptomycin (purchased from Gibco with the product number 15140122), 1 mM sodium pyruvate (purchased from Gibco with the product number 11360-070), 1.5 g/L of sodium bicarbonate (purchased from Sigma with the product number S5761-500G) and 0.1 mM non-essential amino acids. The 0.1 mM non-essential amino acids were prepared by diluting an MEM non-essential amino acid solution (100X) (purchased from Gibco with the product number 11140050) with the MEM at 99 times by volume of the solution.
   3. Trypsin: prepared by diluting 10X trypsin (purchased from Gibco with the product number 15400-054) with DPBS at 9 times by volume of the 10X trypsin.
   4. Assay buffer: prepared by diluting 10X assay buffer (reagent in Mitoscreen flow cytometry mitochondrial membrane potential detection kit, purchased from BD with the product number BDB551302) with DPBS at 9 times the volume of the 10X assay buffer, and placing the assay buffer in a constant-temperature water bath to maintain the temperature at 37°C.
   5. JC-1 acting agent: prepared by dissolving JC-1 dye (reagent in MitoScreen flow cytometry mitochondrial membrane potential detection kit, purchased from BD with the product number BDB551302) in 130 µL of DMSO (purchased from J.T. Baker with the product number 9224-03) to form a JC-1 stock solution and then diluting the JC-1 stock solution and the assay buffer at 99 times the volume of the stock solution.
   6. Flow cytometer, purchased from BD.
B. Test process:
   1. CCD-966SK cells were inoculated at the density of 1×10⁵ per well in a 6-well culture plate containing 2 mL of the cell culture medium per well and cultured at 37°C for 24 hours. Here the CCD-966SK cells were divided into two test groups, namely a blank group and an experimental group respectively. Each group was established in triplicate.
   2. After 24 hours of the culture and attachment of the CCD-966SK cells, the cell culture medium in each group was replaced with an experimental culture medium. The cells in each group were continuously cultured at 37°C for 24 hours. The experimental culture medium of the blank group was the cell culture medium free of a sample; and the experimental culture medium of the experimental group was the cell culture medium containing 0.25% (m/v) of the *Prunus lannesiana* extract prepared in example I.
   3. The experimental culture medium in each group after the culture was removed and the cells were rinsed 2 times with DPBS.
   4. After the rinsing, 200 µL of trypsin was added to each well and reacted for 5 minutes. After the reaction, the cell culture medium was added to terminate the reaction. Then the suspended cells and the cell culture medium in each well were collected into corresponding centrifuge tubes and each centrifuge tube was centrifuged to precipitate the cells.
   5. After a supernatant in the centrifuge tube of each group was removed and the precipitated cells were washed with DPBS 2 times, the cells were resuspended by using 100 µL of JC-1 acting agent, mixed evenly and reacted for 15 minutes in the dark.
   6. After the reaction, the centrifuge tube of each group was centrifuged and the supernatant was removed, and then the precipitated cells were washed 2 times with the assay buffer.
   7. After the washing, 500 µL of DPBS was added to the centrifuge tube of each group to resuspend the cells to form a cell suspension.
   8. After the parameter of an excitation light of the flow cytometer was set to 488 nm and the parameter of an emission light was set to 527 nm and 590 nm, fluorescence signals of each group were detected by the flow cytometer.
C. Test results:
   The relative activity of mitochondria of all groups was calculated according to the following formula: relative activity of mitochondria (%) = (fluorescence signal of each group/fluorescence signal of blank group)×100%.

Statistically significant differences between the test results of the blank group and each of the other groups were obtained by a statistical analysis of a student t-test. In the figure, "*" represented a p-value less than 0.05 when compared to the blank group, "**" represented a p-value less than 0.01 when compared to the blank group, and "***" represented a p-value less than 0.001 when compared to the blank group.

Refer to FIG. 2. The cells in the blank group were not treated with a sample, such that the test results in the blank group represented the performance of the CCD-966SK cells under normal physiological metabolism. Here when the relative activity of mitochondria in the blank group was set to 100%, the relative activity of mitochondria in the experimental group was 110.07%. That is, compared to the blank group, the relative activity of mitochondria in the experimental group was significantly increased by about 10.07% after the *Prunus lannesiana* extract was added to the CCD-966SK cells of the experimental group.

It can be seen from the test results that the *Prunus lannesiana* extract can significantly improve the activity of mitochondria of skin cells. The mitochondria are an electricity generating station of the cells, provide the energy needed by all parts of the body, and play an important role in energy transfer. The higher activity of the mitochondria represented more vigorous physiological activity of the cells. In other words, it is proved by the test results that the *Prunus lannesiana* extract can improve the activity of mitochondria of the cells, thereby enhancing the physiological activity of the cells and further activating skin cells and promoting physiological metabolism.

### Example IV Test for basal metabolic rate

A. Materials and instruments:
   1. Cell line: mouse myoblast cells were purchased from American Type Culture Collection (ATCC) with the cell number CRL-1772^{™} and hereinafter referred to as C2C12 cells.
   2. Cell culture medium: Dulbecco's modified Eagle's medium (DMEM) (purchased from Gibco with the product number of 12100-046) containing 10% fetal bovine serum (purchased from Gibco with the product number 10437-028) and 1% antibiotics. The 1% antibiotics were prepared by diluting 100X antibiotics (Antibiotic-Antimycotic (100X)) (purchased from Gibco with the product number 15240-062) with the DMEM at 99 times by volume of the 100X antibiotics.
   3. Differentiation culture medium: DMEM containing 2% horse serum (purchased from Gibco with the product number 16050-122) and 1% antibiotics.
   4. Pyruvate colorimetric/fluorometric assay kit purchased from BioVision with the product number K609.
   5. Microplate spectrophotometer (ELISA reader) purchased from BioTek company (USA).
B. Test process:
   1. C2C12 cells were inoculated at the density of 1×10⁶ per well in a 6-well culture plate containing 2 mL of the cell culture medium per well and cultured at 37°C until the cell confluence reached eighty percent. Test groups contained a blank group and an experimental group. Each group was established in triplicate.
   2. After the cell confluence reached eighty percent, the cell culture medium in each group was replaced with 2 mL of the differentiation culture medium to induce differentiation of the C2C12 cells into myotubes and the cells were cultured at 37°C until the cells were differentiated into the myotubes.
   3. After the cells were differentiated into the myotubes, the differentiation culture medium in each group was replaced with 2 mL of an experimental culture medium and the cells in each group were continuously cultured at 37°C for 48 hours. The experimental culture medium of the blank group was the differentiation culture medium free of a sample; and the experimental culture medium of the experimental group was the differentiation culture medium containing 0.25% (m/v) of the *Prunus lannesiana* extract prepared in example I.
   4. The experimental culture medium in each group after the culture was removed and the cells were rinsed 1 times with PBS.
   5. After the rinsing, the cells of each group were processed according to the test process provided by the pyruvate colorimetric/fluorometric assay kit to obtain test samples of each group, and then the absorbance at 570 nm (OD₅₇₀ value) of the test samples of each group was measured by using the ELISA reader.
C. Test results:
   The relative content of pyruvate of all groups was calculated according to the following formula: relative content of pyruvate (%) = (OD₅₇₀ value of each group/OD₅₇₀ value of blank group)×100%.

Statistically significant differences between the test results of the blank group and each of the other groups were obtained by a statistical analysis of a student t-test. In the figure, "*" represented a p-value less than 0.05 when compared to the blank group, "**" represented a p-value less than 0.01 when compared to the blank group, and "***" represented a p-value less than 0.001 when compared to the blank group.

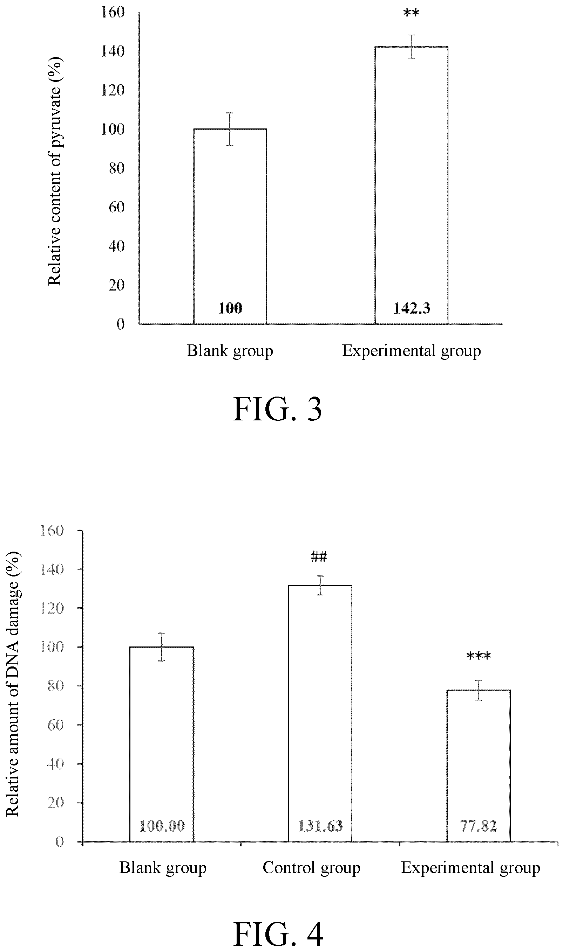

Refer to FIG. 3. The cells in the blank group were not treated with a sample, such that the test results in the blank group represented the performance of the myotubes under normal physiological metabolism. Here when the relative content of pyruvate in the blank group was set to 100%, the relative content of pyruvate in the experimental group was 142.3%. That is, compared to the blank group, the relative content of pyruvate in the experimental group was significantly increased by about 42.3% after the *Prunus lannesiana* extract was added to the myotubes of the experimental group.

It can be seen from the test results that the *Prunus lannesiana* extract can significantly improve the content of pyruvate in the myotubes. Since the physiological metabolism of skeletal muscle cells is one of the main regulators of a basal metabolic rate and pyruvate is an end product of physiological metabolism, the effect of the basal metabolic rate can be evaluated by the content of pyruvate. In other words, it can be proved by the test results that the *Prunus lannesiana* extract can increase the basal metabolic rate of an individual. The *Prunus lannesiana* extract can promote body metabolism, physiological metabolism, cell metabolism, and skin metabolism.

Also, it can be known from example III that the *Prunus lannesiana* extract can promote the activity of mitochondria. It can be known from example IV that the *Prunus lannesiana* extract can increase cell metabolism. Therefore, the *Prunus lannesiana* extract can improve skin activity and accelerate body metabolism, activate skin cells and accelerate metabolism of old and waste cells, and activate and renew skin.

### Example V DNA damage test

A. Materials and instruments:
   1. Cell line: human skin fibroblasts were purchased from the ATCC with the cell number CRL: 1881 and hereinafter referred to as CCD-966SK cells.
   2. Cell culture medium: minimum essential medium (MEM) (purchased from Gibco with the product number 11095080) containing 10% fetal bovine serum (purchased from Gibco with the product number 10437-028) and 1 mM sodium pyruvate (purchased from Gibco with the product number 11360-070).
   3. H₂O₂ solution: prepared from H₂O₂ (purchased from Sigma with the product number 1.08600) and DPBS.
   4.Human phospho-H2Ax (S139) ELISA kit purchased from RayBio with the product number PEL-H2AX-S139-1.
   5. Microplate spectrophotometer (ELISA reader) purchased from BioTek company (USA).
B. Test process:
   1. CCD-966SK cells were inoculated at the density of 2×10⁵ per well in a 6-well culture plate containing 2 mL of the cell culture medium per well and cultured at 37°C for 24 hours. Here the CCD-966SK cells were divided into three test groups, namely a blank group, a control group and an experimental group respectively. Each group was established in triplicate.
   2. After 24 hours of the culture, the cell culture medium in each group was replaced with an experimental culture medium. The experimental culture medium of the blank group and the control group was the cell culture medium free of a sample; and the experimental culture medium of the experimental group was the cell culture medium containing 0.125% (m/v) of the *Prunus lannesiana* extract prepared in example I. Next the cells in each group reacted at 37°C for 1 hour.
   3. After 1 hour of the reaction, a H₂O₂ solution was added in the control group and the experimental group to the final concentration of 2 mM, and the cells in each group reacted at 37°C for 1 hour.
   4. The experimental culture medium in each group after the reaction was removed and the cells were rinsed 1 times with DPBS.
   5. After the rinsing, the cells of each group were processed according to the test process provided by the human phospho-H2Ax (S139) ELISA kit to obtain test samples of each group, and then the absorbance at 450 nm (OD₄₅₀ value) of the test samples of each group was measured by using the ELISA reader.
C. Test results:
   The relative amount of DNA damage of all groups was calculated according to the following formula: relative amount of DNA damage (%) = (OD₄₅₀ value of each group/OD₄₅₀ value of blank group)×100%.

Statistically significant differences between the test results of the blank group and each of the other groups, and between the control group and each of the other groups were obtained by a statistical analysis of a student t-test. In the figure, "#" represented a p-value less than 0.05 when compared to the blank group, "##" represented a p-value less than 0.01 when compared to the blank group, and "###" represented a p-value less than 0.001 when compared to the blank group. Also, in the figure, "*" represented a p-value less than 0.05 when compared to the control group, "**" represented a p-value less than 0.01 when compared to the control group, and "***" represented a p-value less than 0.001 when compared to the control group.

Refer to FIG. 4. The cells in the blank group were not treated with a sample and were not stimulated with H₂O₂, such that the test results in the blank group represented the performance of the cells under normal physiological metabolism. Here when the relative amount of DNA damage in the blank group was set to 100%, the relative amount of DNA damage in the control group was 131.63%, and the relative amount of DNA damage in the experimental group was 77.82%. That is, compared to the blank group, the relative amount of DNA damage in the control group was significantly increased by about 31.63% after the stimulation with H₂O₂ to the cells in the control group. Compared to the control group, the relative amount of DNA damage in the experimental group was significantly reduced by about 40.9% after adding the *Prunus lannesiana* extract and the stimulation with H₂O₂ to the cells in the experimental group. In other words, the difference between the experimental group and the control group was 54% of the relative amount of DNA damage. Compared to the blank group, the relative amount of DNA damage in the experimental group was reduced by about 22.2%.

It can be seen from the test results that the *Prunus lannesiana* extract can significantly reduce the amount of DNA damage of skin cells caused by H₂O₂. H₂O₂ induces the cells to undergo aerobic metabolism, thereby producing ROS. The ROS in the cells attack macromolecules such as nucleic acids, thereby causing DNA damage. In other words, it is proved by the test results that the *Prunus lannesiana* extract can significantly reduce the DNA damage of skin cells caused by oxidation, prevent and/or reduce the DNA damage of the cells, and promote physiological metabolism.

### Example VI Human subject test-blood detection

A. Test process:
   3 healthy adult subjects over 20 years old were administered a bottle of 50 mL of a test drink daily continuously for 4 weeks (i.e. 28 days). The test drink contained 0.7 g of the *Prunus lannesiana* extract prepared in example I and the rest was water. Besides, the subjects underwent blood draw before the administration (hereinafter, referred to as week 0) and after 28 days of the administration (hereinafter, referred to as week 4) to detect the change amount of the concentration of advanced glycation end products (AGEs) in the blood before and after the administration of the *Prunus lannesiana* extract.

In the present example, the concentration of the advanced glycation end products in the blood of the subjects was conducted by external laboratory with reference to the blood test standards published by Ministry of Health and Welfare, Taiwan, and the blood analysis was performed by using an immunoturbidimetric assay.

### B. Test results:

Statistically significant differences between the test results at week 0 and week 4 were obtained by a statistical analysis of a student t-test. In the figure, "*" represented a p-value less than 0.05 when compared to week 0, "**" represented a p-value less than 0.01 when compared to week 0, and "***" represented a p-value less than 0.001 when compared to week 0.

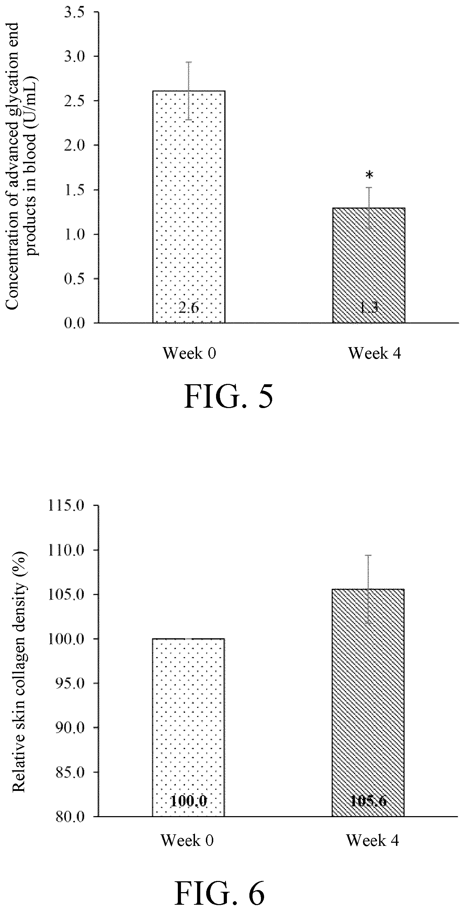

Refer to FIG. 5. FIG. 5 showed the change amount of the concentration of the advanced glycation end products in the blood of the subjects before and after the administration of the *Prunus lannesiana* extract. The concentration of the advanced glycation end products in the blood of the subjects at week 0 was about 2.6 U/mL and the concentration of the advanced glycation end products in the blood of the subjects at week 4 (i.e., after the continuous administration of the *Prunus lannesiana* extract for 4 weeks) was significantly reduced to about 1.3 U/mL. In other words, the concentration of the advanced glycation end products in the blood of the subjects was significantly reduced by 50% after the continuous administration of the *Prunus lannesiana* extract for 4 weeks compared to that before the administration. Besides, the proportion of the population with decreased AGEs was 100%. The advanced glycation end products are nonreducible substances produced by the polymerization of reducing sugars and proteins, and can stimulate the generation of ROS, thereby causing skin aging or dull face. It can be seen from the test results that the *Prunus lannesiana* extract can reduce the concentration of the advanced glycation end products in the blood. The *Prunus lannesiana* extract can reduce the generation of the advanced glycation end products and has an anti-glycation effect. In some embodiments, the *Prunus lannesiana* extract has an effect of preventing skin aging.

### Example VII Human subject test-skin detection

### A. Test process:

10 healthy adult subjects over 20 years old were administered a bottle of 50 mL of a test drink daily continuously for 4 weeks (i.e. 28 days). The test drink contained 0.7 g of the *Prunus lannesiana* extract prepared in example I and the rest was water. Besides, the subjects underwent skin detection and filed in a skin condition questionnaire survey before the administration (faces were clean, hereinafter referred to as week 0) and after 28 days of the administration (faces were clean, hereinafter referred to as week 4). The skin detection was as follows: based on different skin detection items, corresponding instruments and measurement modes were used, numerical values of facial skin were recorded and photographs before and after the administration were taken. The skin detection items were skin collagen density, skin textures, skin wrinkles and skin redness. In addition, at both week 0 and week 4, the temperature and humidity of a test area where the subjects were located were consistent during the detection, so as to reduce the influence of factors such as external temperature and humidity on the skin.

The skin collagen density was detected on the facial skin of the same subjects before the administration and 28 days after the administration by using a high frequency ultrasonic detection probe (High Freq. Ultrasound Module) (DermaLab^{®} USB skin analyzer, Denmark) purchased from Cortex Technology, Denmark. The detection probe transmitted acoustic impulse waves to the skin to convert reflected signals with different intensities into different color patches, the lighter or brighter color represented more collagen content in the skin, and the color patches were calculated to obtain a value (hereinafter referred to as skin collagen density value) representing the skin collagen density. Here the higher obtained skin collagen density value indicated the higher skin collagen density. Then the relative skin collagen density was calculated according to the following formula: relative skin collagen density (%) = (skin collagen density value of each group/skin collagen density value before administration)×100%.

The skin texture was detected on the facial skin of the same subjects before the administration and 28 days after the administration by using VISIA Complexion Analysis System purchased from Canfield Scientific, USA. The analysis system photographed the facial skin through a high-resolution camera lens and obtained a value (hereinafter referred to as skin texture degree value) representing the roughness of the skin through analysis and calculation by a standard white light irradiation and detection of skin pits and projections. Here the higher obtained skin texture degree value indicated the higher skin roughness degree. Then the relative skin texture degree was calculated according to the following formula: relative skin texture degree (%) = (skin texture degree value of each group/skin texture degree value before administration)×100%.

The skin wrinkles were detected on the facial skin of the same subjects before the administration and 28 days after the administration by using VISIA Complexion Analysis System purchased from Canfield Scientific, USA. The analysis system photographed the facial skin through a high-resolution camera lens and obtained a value (hereinafter referred to as skin wrinkle degree value) representing the wrinkle degree of the skin through analysis and calculation of the lengths and depths of the wrinkles by a standard white light irradiation and detection of the changes of skin shadows. Here the higher obtained skin wrinkle degree value indicated the higher skin wrinkle degree. Then the relative skin wrinkle degree was calculated according to the following formula: relative skin wrinkle degree (%) = (skin wrinkle degree value of each group/skin wrinkle degree value before administration)×100%.

The skin redness was detected on the facial skin of the same subjects before the administration and 28 days after the administration by using VISIA Complexion Analysis System purchased from Canfield Scientific, USA. The analysis system photographed the facial skin through a RBX polarized light technology and obtained a value (hereinafter referred to as skin redness degree value) representing the redness condition of the skin by detecting skin deep blood vessels or hemoglobin. Here the higher obtained skin redness degree value indicated the serious skin redness condition. Then the relative skin redness degree was calculated according to the following formula: relative skin redness degree (%) = (skin redness degree value of each group/skin redness degree value before administration)×100%.

The skin condition questionnaire survey was that the subjects performed self-assessment by using a skin condition questionnaire which was shown in table I below. The subjects self-assessed skin conditions (including more stable overall skin condition, compact skin, fewer fine lines around the eyes, relieved sallow skin, glossy and bright skin, tender and smooth skin, less tendency to redness and sensitivity, and skin moisture). Each subject self-assessed the skin conditions, determined the agreement degree of the skin conditions and selected one of these four options, namely strongly agree, agree, no opinion and disagree.

**Table I**

| Agreement degree | Strongly agree | Agree | No opinion | Disagree |
|---|---|---|---|---|
| More stable overall skin condition | | | | |
| Compact skin | | | | |
| Fewer fine lines around the eyes | | | | |
| Relieved sallow skin | | | | |
| Glossy and bright skin | | | | |
| Tender and smooth skin | | | | |
| Less tendency to redness and sensitivity | | | | |
| Skin moisture | | | | |

The skin condition questionnaire survey was that the subjects performed self-assessment by using an efficacy satisfaction degree questionnaire which was shown in table II below. The subjects self-assessed the efficacy satisfaction degree. Each subject self-assessed the skin conditions, determined the efficacy satisfaction degree and selected one of these four options, namely strongly satisfied, satisfied, no opinion and dissatisfied. The higher satisfaction degree indicated the better self- assessment of the overall skin conditions.

**Table II**

| Satisfaction degree | Strongly satisfied | Satisfied | No opinion | Dissatisfied |
|---|---|---|---|---|
| Efficacy satisfaction degree | | | | |

### B. Test results:

Statistically significant differences between the test results at week 0 and week 4 were obtained by a statistical analysis of a student t-test. In the figures, "*" represented a p-value less than 0.05 when compared to week 0, "**" represented a p-value less than 0.01 when compared to week 0, and "***" represented a p-value less than 0.001 when compared to week 0.

### 1. Detection results of skin collagen density of subjects

Refer to FIG. 6. The skin collagen density detected in 10 subjects before the administration was taken as the relative skin collagen density of 100%. At this time, the average relative skin collagen density at week 4 (i.e., after the continued administration of the *Prunus lannesiana* extract for 4 weeks) was 105.6%. In other words, the relative skin collagen density of the subjects was increased by 5.6% after the continuous administration of the *Prunus lannesiana* extract for 4 weeks compared to that before the administration. Besides, the proportion of the improved population was 70%. Therefore, the *Prunus lannesiana* extract can increase collagen, increase the collagen content of the skin and effectively promote the collagen proliferation, thereby further providing the elasticity and flexibility of the skin, enabling the skin to be elastic and further improving the skin conditions of the subjects. The *Prunus lannesiana* extract has the effects of promoting the generation of skin collagen, increasing the skin collagen density and enabling the skin to be elastic, fine and glossy.

### 2. Detection results of skin texture of subjects

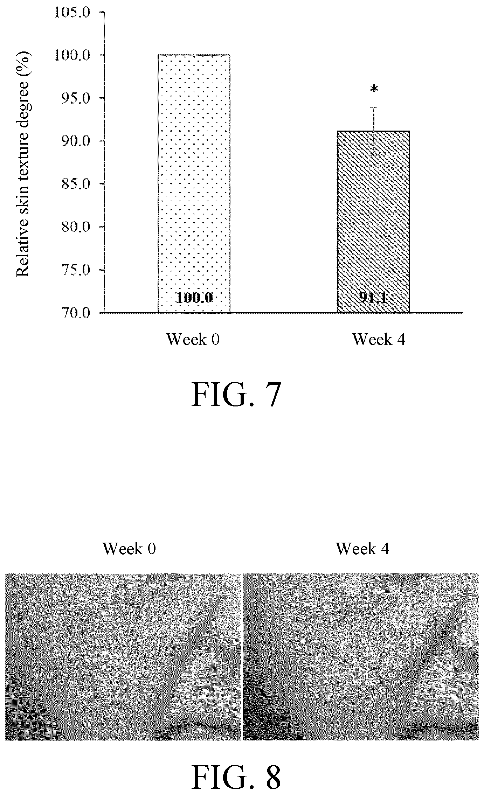

Refer to FIG. 7. The skin texture condition detected in 10 subjects before the administration was taken as the relative skin texture degree of 100%. At this time, the average relative skin texture degree at week 4 (i.e., after the continued administration of the *Prunus lannesiana* extract for 4 weeks) was 91.1%. In other words, the relative skin texture degree of the subjects was significantly reduced by 8.9% after the continuous administration of the *Prunus lannesiana* extract for 4 weeks compared to that before the administration. Besides, the proportion of the improved population was 90%.

Refer to FIG. 8. FIG. 8 showed photographs of the skin textures of one of 10 subjects at week 0 and week 4. The subject had obviously less skin textures (yellow green marks) and obviously smoother skin at week 4 compared to those at week 0. Therefore, the *Prunus lannesiana* extract can smooth the skin textures, decrease and/or reduce skin roughness, enable the skin to be smoother, and improve the skin conditions of the subjects.

### 3. Detection results of skin wrinkles of subjects

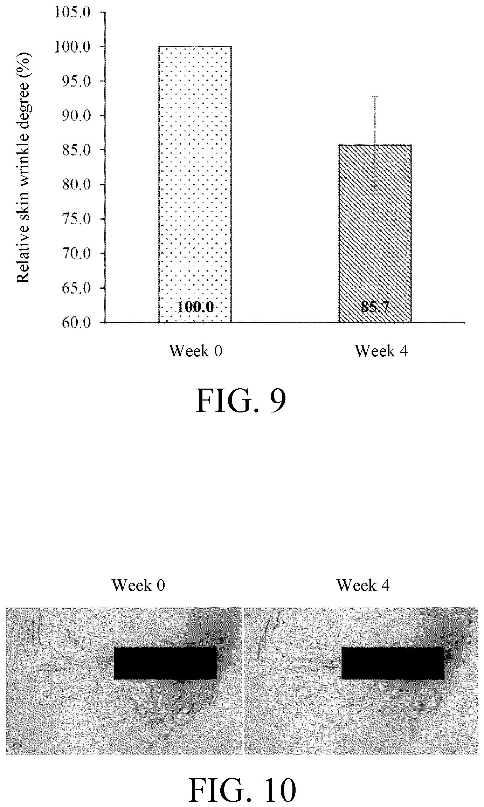

Refer to FIG. 9. The skin wrinkle condition detected in 10 subjects before the administration was taken as the relative skin wrinkle degree of 100%. At this time, the average relative skin wrinkle degree at week 4 (i.e., after the continued administration of the *Prunus lannesiana* extract for 4 weeks) was 85.7%. In other words, the relative skin wrinkle degree of the subjects was reduced by 14.3% after the continuous administration of the *Prunus lannesiana* extract for 4 weeks compared to that before the administration. Besides, the proportion of the improved population was 70%.

Refer to FIG. 10. FIG. 10 showed photographs of fine lines around the eyes of one of 10 subjects at week 0 and week 4. The subject had obviously fewer and shorter fine lines around the eyes at week 4 compared to those at week 0. Therefore, the *Prunus lannesiana* extract can reduce and smooth skin wrinkles and/or fine lines, and improve the skin conditions of the subjects.

### 4. Detection results of skin redness of subjects

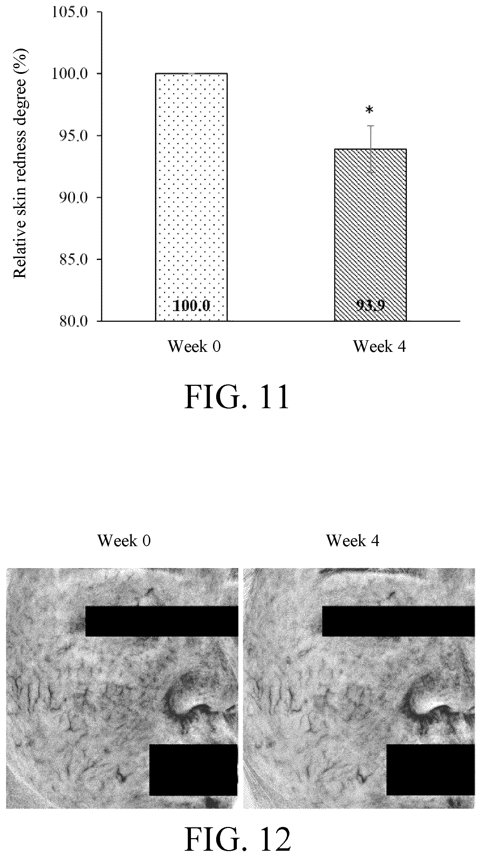

Refer to FIG. 11. The skin redness condition detected in 10 subjects before the administration was taken as the relative skin redness degree of 100%. At this time, the average relative skin redness degree at week 4 (i.e., after the continued administration of the *Prunus lannesiana* extract for 4 weeks) was 93.9%. In other words, the relative skin redness degree of the subjects was significantly reduced by 6.1% after the continuous administration of the *Prunus lannesiana* extract for 4 weeks compared to that before the administration. Besides, the proportion of the improved population was 80%.

Refer to FIG. 12. FIG. 12 showed photographs of the skin redness of one of 10 subjects at week 0 and week 4. The subject had obviously relieved skin redness at week 4 compared to that at week 0. Therefore, the *Prunus lannesiana* extract can relieve skin redness, reduce and/or relieve skin redness, and improve the skin conditions of the subjects.

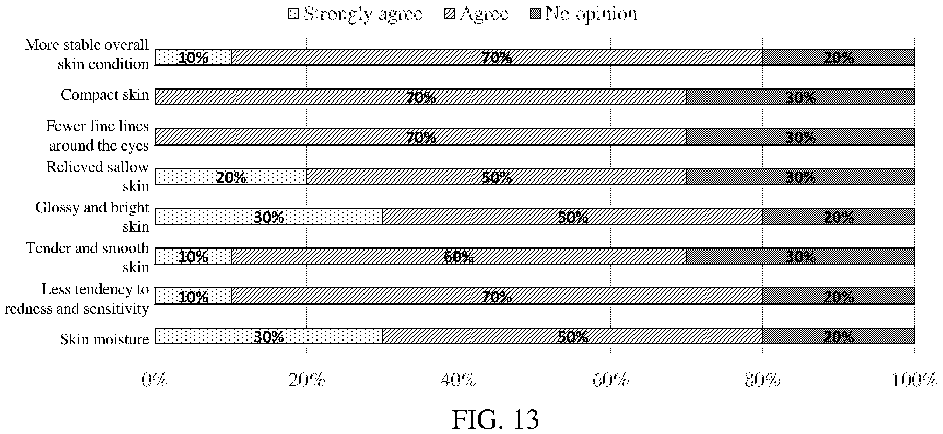

5. The results of the self-assessment of "skin conditions" were shown in FIG. 13. 10 subjects self-assessed the skin conditions at week 4 (i.e., after the continued administration of the *Prunus lannesiana* extract for 4 weeks). For the more stable overall skin condition, 10% of the subjects strongly agreed, 70% of the subjects agreed, 20% of the subjects had no opinion and 0% of the subjects disagreed. For the compact skin, 0% of the subjects strongly agreed, 70% of the subjects agreed, 30% of the subjects had no opinion and 0% of the subjects disagreed. For the fewer fine lines around the eyes, 0% of the subjects strongly agreed, 70% of the subjects agreed, 30% of the subjects had no opinion and 0% of the subjects disagreed. For the relieved sallow skin, 20% of the subjects strongly agreed, 50% of the subjects agreed, 30% of the subjects had no opinion and 0% of the subjects disagreed. For the glossy and bright skin, 30% of the subjects strongly agreed, 50% of the subjects agreed, 20% of the subjects had no opinion and 0% of the subjects disagreed. For the tender and smooth skin, 10% of the subjects strongly agreed, 60% of the subjects agreed, 30% of the subjects had no opinion and 0% of the subjects disagreed. For the less tendency to redness and sensitivity, 10% of the subjects strongly agreed, 70% of the subjects agreed, 20% of the subjects had no opinion and 0% of the subjects disagreed. For the skin moisture, 30% of the subjects strongly agreed, 50% of the subjects agreed, 20% of the subjects had no opinion and 0% of the subjects disagreed.

In other words, the subjects had the stable overall skin condition, compact skin, reduced fine lines around the eyes, relieved sallow skin, improved glossy and bright skin, improved tender and smooth skin, reduced redness and sensitivity, and improved skin moisture after the continuous administration of the *Prunus lannesiana* extract for 4 weeks compared to those before the administration. Besides, 70% to 80% of the subjects considered that the above-mentioned skin conditions were improved. Therefore, the *Prunus lannesiana* extract can improve stability of an overall skin condition, improve skin tightness, reduce fine lines around the eyes, relieve sallow skin, improve glossy and bright skin, improve tender and smooth skin, relieve skin redness and sensitivity, and improve skin moisture, and further improve the skin condition of the subjects.

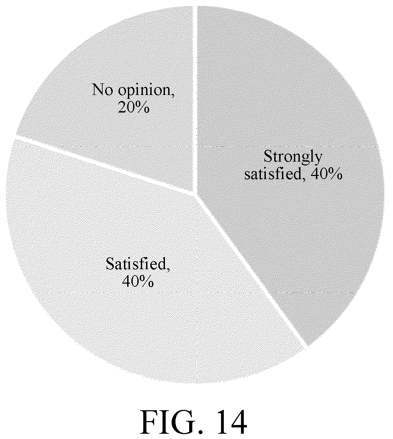

6. The results of the self-assessment of "efficacy satisfaction degree" were shown in FIG. 14. 10 subjects self-assessed the efficacy satisfaction degree at week 4 (i.e., after the continued administration of the *Prunus lannesiana* extract for 4 weeks). For the efficacy satisfaction degree, 40% of the subjects were strongly satisfied, 40% of the subjects were satisfied, 20% of the subjects had no opinion and 0% of the subjects were dissatisfied.

In other words, the overall skin condition of the subjects can be improved after the continuous administration of the *Prunus lannesiana* extract for 4 weeks compared to that before the administration. Besides, up to 80% of the subjects were satisfied with the efficacy of the *Prunus lannesiana* extract. Therefore, the *Prunus lannesiana* extract can improve the overall skin condition.

### INDUSTRIAL APPLICATION

In summary, use of the *Prunus lannesiana* extract of any embodiments in improving skin condition or basal metabolic rate relates to the use of the *Prunus lannesiana* extract for preparing a composition for improving skin condition or basal metabolic rate, thereby providing the composition capable of improving the skin condition or the basal metabolic rate on an individual when administered to the individual. In other words, the composition has functions of improving the skin condition or the basal metabolic rate. In some embodiments, the composition prepared from the *Prunus lannesiana* extract further has one or more of the following functions: improving skin collagen density, reducing skin wrinkles and/or fine lines, relieving skin redness, slowing down skin aging or oxidation, decreasing skin roughness, improving stability of skin condition, improving skin tightness, relieving sallow skin, improving glossy and bright skin, improving tender and smooth skin, relieving skin sensitivity, improving skin moisture, improving activity of mitochondria, preventing and/or reducing DNA damage, improving antioxidant activity, and reducing generation of advanced glycation end products.

## Claims

1. Use of a *Prunus lannesiana* extract for preparing a composition for improving a skin condition, wherein the *Prunus lannesiana* extract is obtained by drying *Prunus lannesiana* and extracting the dried *Prunus lannesiana* with a solvent at a weight ratio of the solvent to the *Prunus lannesiana* of (20-40): 1.

2. The use according to claim 1, wherein the *Prunus lannesiana* extract improves a skin collagen density.

3. The use according to claim 1, wherein the *Prunus lannesiana* extract reduces skin wrinkles and/or fine lines.

4. The use according to claim 1, wherein the *Prunus lannesiana* extract relieves skin redness.

5. The use according to claim 1, wherein the *Prunus lannesiana* extract slows down skin aging or oxidation.

6. The use according to claim 1, wherein the *Prunus lannesiana* extract decreases skin roughness, improves stability of skin condition, improves skin tightness, relieves sallow skin, improves glossy and bright skin, improves tender and smooth skin, relieves skin sensitivity or improves skin moisture.

7. Use of a *Prunus lannesiana* extract for preparing a composition for improving a basal metabolic rate, wherein the *Prunus lannesiana* extract is obtained by drying *Prunus lannesiana* and extracting the dried *Prunus lannesiana* with a solvent at a weight ratio of the solvent to the *Prunus lannesiana* of (20-40): 1.

8. The use according to claim 7, wherein the *Prunus lannesiana* extract improves activity of mitochondria.

9. The use according to claim 7, wherein the *Prunus lannesiana* extract prevents and/or reduces DNA damage.

10. The use according to claim 7, wherein the *Prunus lannesiana* extract improves an antioxidant activity.

11. The use according to claim 7, wherein the *Prunus lannesiana* extract reduces generation of advanced glycation end products.
